# EUROPÄISCHE PATENTSCHRIFT

(11) **EP 3 558 094 B1**
(45) Veröffentlichungstag und Bekanntmachung des Hinweises auf die Patenterteilung: **22.07.2026**
(21) Anmeldenummer: 17825421.5
(22) Anmeldetag: 12.12.2017
(51) Int. Cl.: A61B 5/00

(54) **VERFAHREN UND ANORDNUNG ZUM ERMITTELN EINES SCHÄDIGUNGSGRADS VON HAAR**
METHOD AND ARRANGEMENT FOR DETERMINING A DEGREE OF DAMAGE TO HAIR
PROCÉDÉ ET DISPOSITIF PERMETTANT DE DÉTERMINER UN DEGRÉ DE DÉTÉRIORATION DES CHEVEUX

(30) Priorität: 20.12.2016 DE 102016225674
(43) Veröffentlichungstag der Anmeldung: 30.10.2019
(73) Patentinhaber: Henkel AG & Co. KGaA, 40589 Düsseldorf (DE)
(72) Erfinder: MÜLLER, Burkhard, 40221 Düsseldorf (DE); LECHNER, Torsten, 40764 Langenfeld (DE)
(86) Internationale Anmeldenummer: PCT/EP2017/082352
(87) Internationale Veröffentlichungsnummer: WO 2018/114459

(56) Entgegenhaltungen:
- US-A1- 2013 287 715
- MANUEL GAMEZ-GARCIA: "The effects of lipid penetration and removal from subsurface microcavities and cracks at the human cuticle sheath", J. COSMET. SCI, 60, 1 April 2009 (2009-04-01), pages 85 - 95, XP055458042, Retrieved from the Internet <URL:http://www.nononsensecosmethic.org/wp-content/uploads/2015/04/The-effects-of-lipid-penetration-and-removal-from-subsurface-microcavities.pdf> [retrieved on 20180309]
- MANUEL GAMEZ-GARCIA ET AL: "Patterns of light interference produced by damaged cuticle cells in human hair", JOURNAL OF THE SOCIETY OF COSMETIC CHEMISTS, VOL. 58, NO. 4, 269-282, 1 January 2007 (2007-01-01), pages abstract, XP055458010, Retrieved from the Internet <URL:http://journal.scconline.org/abstracts/cc2007/cc058n04/p00269-p00282.html> [retrieved on 20180309]
- SANGYUN LEE ET AL: "Quantitative Morphological and Biochemical Studies on Human Downy Hairs using 3-D Quantitative Phase Imaging", 15 May 2015 (2015-05-15), XP055458012, Retrieved from the Internet <URL:https://arxiv.org/ftp/arxiv/papers/1505/1505.04231.pdf>

## Beschreibung

Die Erfindung betrifft Verfahren und Anordnungen zum Ermitteln eines Schädigungsgrads von Haar.

Bei einer Behandlung von Haar mit kosmetischen Produkten kann eine Wirkung des Produkts, z.B. eine Intensität einer Färbung, stark vom Schädigungsgrad des Haars abhängen. Deshalb kann die Ermittlung einer Schädigung des Haars von großer Bedeutung sein. Außerdem kann es wünschenswert sein, einer Schädigung von Haar entgegenzuwirken, wobei wiederum eine Ermittlung des Schädigungsgrads nützlich ist, um ein geeignetes Pflegeprodukt auswählen zu können. Dementsprechend sind effektive und für den Benutzer bequeme Möglichkeiten zur Ermittlung des Schädigungsgrads von Haar wünschenswert.

Dokumente "The effects of lipid penetration and removal from subsurface microcavities and cracks at the human cuticle sheath" von Manuel Gamez.Garcia, J Cosmet. Sci., 60, 85-95 (März/April 2009) und Dokument "Patterns of Light Interference Produced by Damaged Cuticle Cells in Human Hair" von Manuel Gamez-Garcia und Yuan Lu, Journal of the Society of Cosmetic Chemists, Bd. 58, Nr. 4, 269-282 beschreiben quantitative optische Experimente, die an menschlichen Armhaaren durchgeführt wurden. Dokument US2013287715 A1 beschreibt kosmetische Zusammensetzungen, die hydrolysierte Hefeproteine umfassen, und deren Verwendung als kosmetische Zusammensetzungen.

Gemäß verschiedenen Ausführungsbeispielen wird ein Verfahren gemäß Anspruch 1 beschrieben. Es geht um ein verfahren zum Ermitteln eines Schädigungsgrads von Haar, das aufweist: Während eines Belichtens einer Haarprobe des Haars mit Licht, Registrieren von Licht, welches von der Haarprobe abgestrahlt wird, Ermitteln, basierend auf dem registrierten Licht, von ersten Bereichen der Haarprobe, die das Licht mit höherer Interferenz reflektieren und zweiten Bereichen der Haarprobe, die das Licht mit niedrigerer Interferenz reflektieren und Ermitteln eines Schädigungsgrads der Haarprobe basierend auf den Größen der ersten Bereiche und der zweiten Bereiche.

Gemäß einem weiteren Ausführungsbeispiel wird eine Anordnung zum Ermitteln eines Schädigungsgrads von Haar gemäß dem oben beschriebenen Verfahren bereitgestellt.

Ausführungsbeispiele der Erfindung sind in den Figuren dargestellt und werden im Folgenden näher erläutert.
Figur 1 illustriert die Entnahme einer Haarprobe. Figur 2 zeigt den Aufbau eines Haares.
Figur 3 zeigt eine Anordnung zur Ermittlung eines Schädigungsgrads von Haar.
Figur 4 zeigt ein Beispiel für ein Bild von einem einzelnen Haar, das gemäß einem Ausführungsbeispiel aufgenommen wird.
Figur 5 zeigt eine Kurve, die ein Beispiel für den Zusammenhang zwischen einem HaarSchädigungsgrad und dem Anteil heller Flächen im Interferenzbild darstellt.
Figur 6 zeigt ein Ablaufdiagramm, das ein Verfahren zum Ermitteln eines Schädigungsgrads von Haar veranschaulicht.
Figur 7 zeigt eine Anordnung zum Ermitteln eines Schädigungsgrads von Haar

In der folgenden ausführlichen Beschreibung wird auf die beigefügten Zeichnungen Bezug genommen, die einen Teil der vorliegenden Anmeldung bilden und in denen zur Veranschaulichung spezifische Ausführungsformen gezeigt sind, in denen die Erfindung ausgeübt werden kann. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

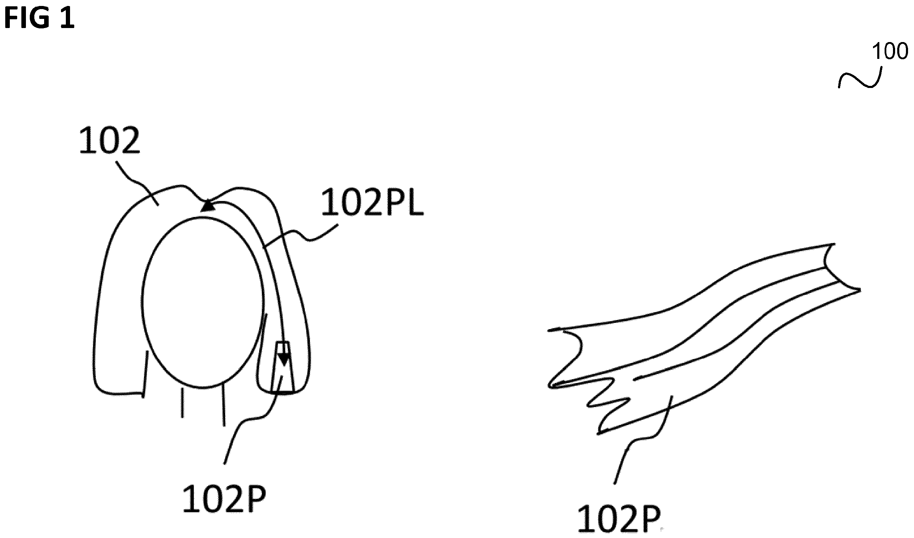

Figur 1 illustriert die Entnahme einer Haarprobe.

Gemäß verschiedenen Ausführungsbeispielen kann zum Ermitteln eines Schädigungsgrads von Haar 102 eines Nutzers eine Haarprobe 102P entnommen werden. Die Haarprobe 102P kann beispielsweise in einer Entfernung 102PL von einer Kopfhaut des Nutzers entnommen werden und kann ein einzelnes oder mehrere Haare aufweisen.

Ein Ermitteln des Schädigungsgrads von Haar 102 eines Nutzers kann auch ohne ein Entnehmen einer Haarprobe 102P direkt am Kopf des Nutzers erfolgen.

Im Folgenden werden Ausführungsbeispiele beschrieben, bei denen eine Schädigung von Haar mittels Interferenzreflexionsmikroskopie durchgeführt wird.

Mit Hilfe der Interferenzreflexionsmikroskopie können sehr dünne Haarstrukturen untersucht werden. Die Interferenzmikroskopie beruht auf der Bildung von Interferenzen, die entstehen, wenn Licht an der oberen und der unteren Grenzfläche einer Struktur reflektiert wird und reflektiertes Licht von beiden Grenzflächen miteinander interferiert. Dadurch entstehen Interferenzmuster, die beobachtet werden können, und die Aufschluss über die Dicke der Struktur liefern. Die entstehenden Interferenzfarben lassen Strukturmessungen im Bereich unter 200 nm zu. Durch Betrachten der Interferenzfarben durch ein Lichtmikroskop können diese Strukturmessungen entsprechend zu mikroskopisch erkennbaren Strukturen zugeordnet werden.

Gemäß verschiedenen Ausführungsformen wird dies auf die Cuticula eines Haares angewendet.

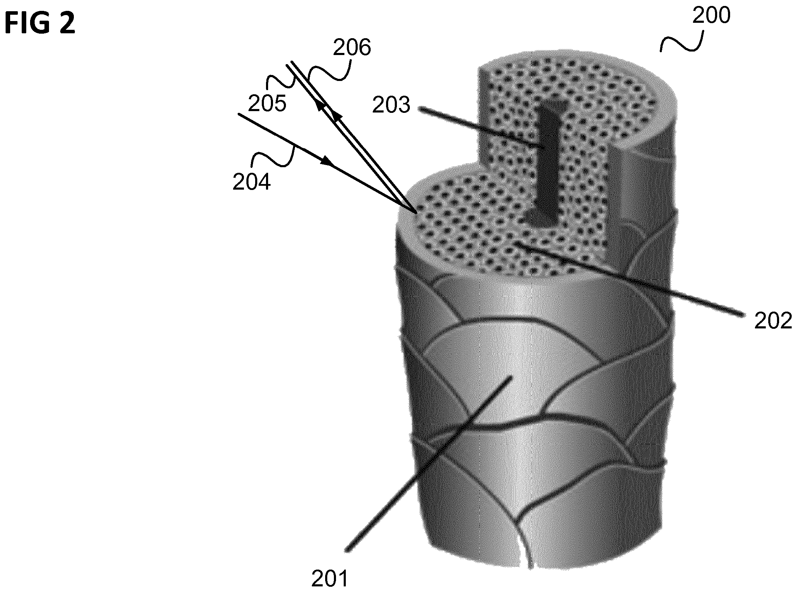

Figur 2 zeigt den Aufbau eines Haares 200.

Das Haar weist eine Cuticula 201, einen Kortex 202 und Mark 203 auf.

Wird das Haar mit Licht 204 bestrahlt, so wird ein Teil des Lichts an der Außenfläche der Cuticula 201 reflektiert und ein Teil wird an der Grenzfläche zwischen Cuticula 201 und Kortex 202 reflektiert (insbesondere dann, wenn die Cuticula 201 sich von dem Kortex 202 abgehoben oder abgelöst hat, was typischerweise einer Schädigung des Haares entspricht). Die reflektierten Teile 205, 206 interferieren und bilden ein Interferenzmuster.

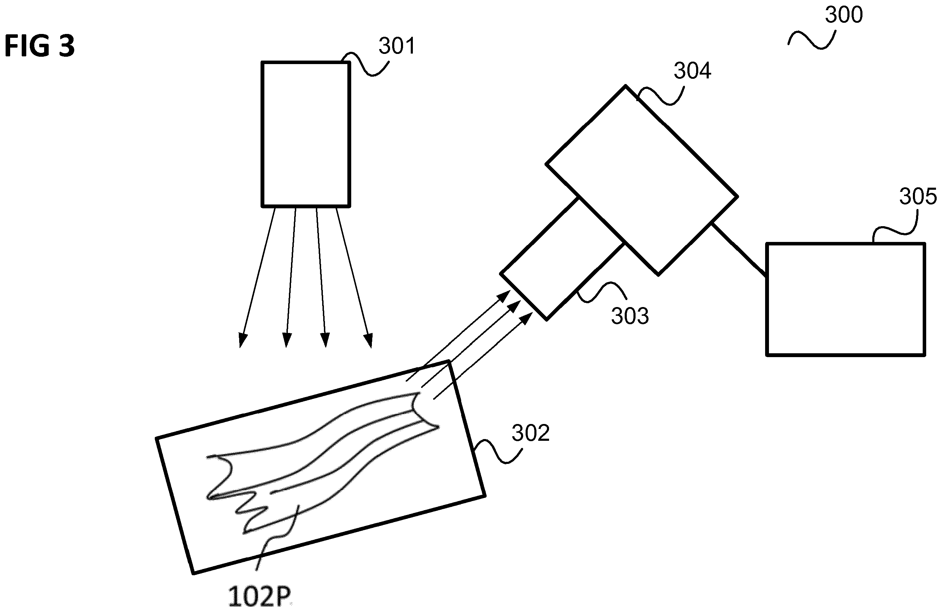

Figur 3 zeigt eine Anordnung 300 zur Ermittlung eines Schädigungsgrads von Haar.

Die Anordnung weist eine Lichtquelle 301 auf, die derart angeordnet ist, dass sie eine Haarprobe 102P, die beispielsweise mehrere Haare (d.h. Haarfasern) aufweist, mit Weißlicht bestrahlt.

Die Haarprobe 102P ist beispielsweise in oder auf einem Träger 302 angeordnet. Eine Kamera 304, die an ein Interferenzmikroskop 303 gekoppelt ist, das auf die Haarprobe 102P gerichtet ist und das von der Haarprobe 102P reflektierte Licht empfängt, erstellt Fotos von ein oder mehreren Haarfasern.

Der Vergrößerungsfaktor des Mikroskops liegt beispielsweise im Bereich von 10 bis 1000, z.B. im Bereich 200 bis 400 und ist beispielsweise einstellbar.

Die Anordnung 300 weist ferner eine Datenverarbeitungseinrichtung 305 auf, die eingerichtet ist, die Fotos auszuwerten und basierend auf den Fotos eine Schädigung (z.B. einen Schädigungsgrad) zu ermitteln.

Die Datenverarbeitungseinrichtung 305 bestimmt beispielsweise für jedes von ein oder mehreren Fotos Art und/oder Anzahl der Interferenz-Muster der Haare mittels einer Bildanalyse-Software, vergleicht sie mit einem auf gleiche Art erstellten Kalibriermodell und bestimmt so einen Schädigungsgrad der Haare. Bei mehreren Fotos kann die Datenverarbeitungseinrichtung 305 beispielsweise einen Mittelwert der für die Fotos ermittelten Schädigungsgrade bilden.

Das Interferenzmikroskop 303, die Kamera 304, die Datenverarbeitungseinrichtung 305 und gegebenenfalls auch die Lichtquelle 301 und der Träger 302 können durch ein Smartphone realisiert werden, das mit einem Mikroskop-Objektiv für Smartphones ausgestattet ist, das auch für Interferenzmikroskopie geeignet ist. Alternativ können das Interferenzmikroskop 303, die Kamera 304, die Datenverarbeitungseinrichtung 305 und gegebenenfalls auch die Lichtquelle 301 und der Träger 302 durch einen WLAN-fähigen Mediaplayer wie dem iPod touch oder ein Tablet realisiert werden.

Beispielsweise werden mittels eines portablen elektronischen Geräts (wie beispielsweise eines Smartphones, eines Tablets, etc.) mit Mikroskop-Aufsatz (wie beispielsweise einem Scrona µpeek) in Kombination mit einem Interferenz-Schieber (engl. interference slider, wie beispielsweise angeboten von Hirox Ltd.) Haare bei 350fachen Vergrößerung aufgenommen.

Die Interferenzfarben werden durch ein Lichtmikroskop hindurch beobachtet und deshalb können diese Strukturmessungen den mikroskopisch erkennbaren Strukturen zugeordnet werden. Um Kontrast vermindernde Reflexionen an Glasoberflächen (z.B. des Trägers 302) zu minimieren, können eine Ölimmersion und ein reflexionsarmes Objektiv verwendet werden. Eine Zentralblende kann im Beleuchtungsstrahlengang angeordnet werden, um Reflexionen aus dem zentralen Bereich des Objektivs zu blockieren.

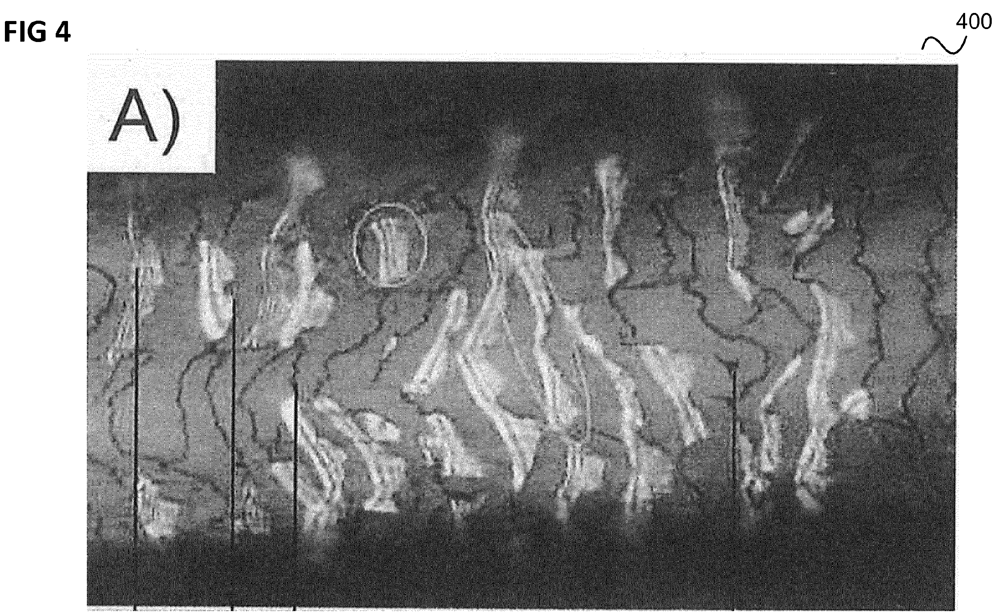

Figur 4 zeigt ein Beispiel für ein Bild 400 von einem einzelnen Haar, das beispielsweise mittels der Kamera 304 registriert (d.h. aufgenommen) wird.

Das Bild 400 kann von der Kamera 304 in Farbe aufgenommen werden und von der Datenverarbeitungseinrichtung 305 in eine Schwarzweiß-Version umgewandelt werden, wie sie Figur 4 zeigt. Alternativ kann die Kamera 304 das Bild 400 in Schwarzweiß aufnehmen.

Die Datenverarbeitungseinrichtung kann den Flächenanteil von hellen Flächen in dem Bild 400 ermitteln, beispielsweise mit einem auf der Datenverarbeitungseinrichtung installierten Bildverarbeitungscomputerprogramm. Dazu kann ein Helligkeitsschwellwert vorgegeben werden und die Datenverarbeitungseinrichtung erkennt Flächen mit einer geringeren Helligkeit als dem Helligkeitsschwellwert als dunkel und Flächen mit einer höheren Helligkeit als dem Helligkeitsschwellwert als hell. Der Helligkeitsschwellwert ist beispielsweise mit Bezug auf die Helligkeit der dunklen Bereiche des Fotos definiert (z.B. als Faktor der Helligkeit der dunklen Bereiche).

Der Flächenteil der interferierenden Haarstrukturen, das heißt der Anteil der hellen Bereiche, an dem Gesamtbereich des Haars in dem Foto, liegt beispielsweise zwischen 1% und 50%, z.B. zwischen 5% und 30%.

Die Datenverarbeitungseinrichtung kann dann von dem ermittelten Flächenanteil heller Flächen auf den Schädigungsgrad schließen, beispielsweise mit Hilfe einer Tabelle, die Bereiche von Flächenanteilen Schädigungsgraden zuordnet.

Zur Kalibrierung (d.h. beispielsweise zum Erstellen einer solchen Tabelle) werden beispielsweise Haare von Haarproben durch eine vorgegebene Zugdehnung der Haare gedehnt und damit geschädigt. Beispielsweise wird der Dehnungsgrad für alle Haarproben konstant gehalten und die Anzahl an Dehnungs-/Entspannungszyklen variiert, um verschiedene Schädigungsgrade zu erzielen, zum Beispiel im Bereich von 1 bis 1000 Dehnungs-Entspannungszyklen. Die (je nach Anzahl von Dehnungs-Entspannungszyklen) resultierenden Haarschädigungen werden wie oben beschrieben mittels Interferenzmikroskopie untersucht und mit einer Bildbearbeitungs-Software, die den Flächenanteil des interferierenden Haaroberflächen (welche deutlich heller als Rest des Haares dargestellt werden) bestimmt, analysiert. Die ermittelten Flächenanteile sind ein Maß für den Haarschädigungsgrad und können den Schädigungsgraden (die beispielsweise Bereichen von Anzahlen von Dehnungs-/Entspannungszyklen entsprechen) zugeordnet werden, um die Datenverarbeitungseinrichtung zu kalibrieren.

Beispielsweise werden zur Kalibrierung Haare geschädigt, indem sie bei Raumtemperatur um 20% gedehnt und dann für 1 Sekunde entspannt werden. Dieser Prozess wird mehrfach durchgeführt. Zum Beispiel werden je 10 Haare den folgenden Anzahlen von Dehnungen ausgesetzt:
- 1
- 3
- 10
- 30
- 100
- 300
- 1000

Anschließend wurden alle Haare einer Gruppe (d.h. die Haare, die derselben Anzahl von Dehnungen ausgesetzt wurden) mikroskopiert und mit einer Bildanalysen-Software der Anteil heller (interferierender) Flächen bestimmt und anschließend der Durchschnitt über die Haare der Gruppe bestimmt. Auf diese Weise können Flächenanteile den erzeugten Haarschädigungen zugeordnet werden.

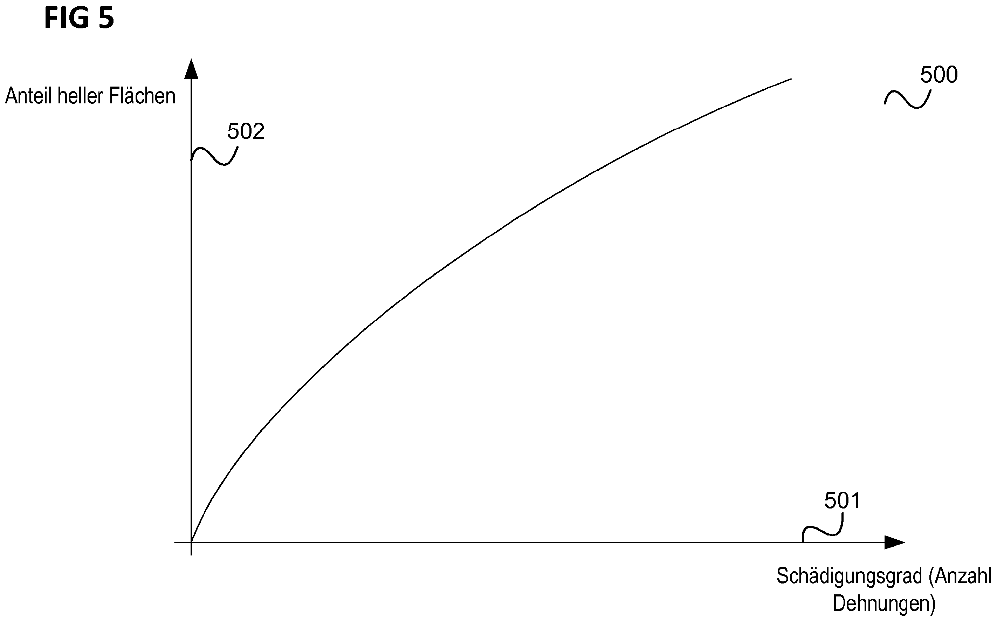

Durch Interpolation des Anteils heller Flächen für die verschiedenen Gruppen kann für jeden Anteil von hellen Flächen ein Schädigungsgrad - ausgedrückt als Anzahl an Dehnungen um 20% - ermittelt werden, so dass sich beispielsweise eine Kurve ergibt, wie sie in Figur 5 dargestellt ist.

Figur 5 zeigt eine Kurve 501, die ein Beispiel für den Zusammenhang zwischen einem HaarSchädigungsgrad, zunehmend von links nach rechts entlang der x-Achse 502, und dem Anteil heller Flächen im Interferenzbild, zunehmend von unten nach oben entlang der y-Achse 503, darstellt.

Der Schädigungsgrad wird beispielsweise wie in Tabelle 1 dargestellt Schädigungsstufen zugeordnet.

**Tabelle 1**

| Schädigungsgrad als Anzahl Dehnungen | Schädigungsstufe |
|---|---|
| 0 bis 3 | Gering |
| 4 bis 10 | Mäßig |
| 11 bis 100 | Stark |
| mehr als 100 | Sehr stark |

Es können beispielsweise 2 bis 20 Schädigungsstufen definiert werden, z.B. 2 bis 4.

Die Haarschädigung von Testhaaren (d.h. Haaren, deren Schädigungsgrad zu bestimmen ist) wird gemäß der Kalibrierkurve, wie sie beispielsweise in Figur 5 dargestellt ist und beispielsweise in Tabellenform (z.B. online) hinterlegt ist, bestimmt. Aus der Haarschädigung (beispielsweise für jede Schädigungsstufe gemäß Tabelle 1) leitet die Datenverarbeitungseinrichtung 305 eine Produktempfehlung ab, beispielsweise basierend auf Erfahrungswerten, z.B. auf Basis von Erfahrungstabellen, und schlägt sie dem Benutzer vor. Wird die Datenverarbeitungseinrichtung 305 durch ein Smartphone oder Tablet realisiert, zeigt das Smartphone oder das Tablet beispielsweise dem Benutzer eine Produktempfehlung auf seinem Display an. Alternativ kann im Fall, dass die Datenverarbeitungseinrichtung 305 durch ein Smartphone oder Tablet realisiert ist, dem Benutzer eine Produktempfehlung mittels eines Lautsprechers angesagt werden.

Beispielsweise empfiehlt die Datenverarbeitungseinrichtung 305 für stärker geschädigte Haare Produkte mit höherer Pflegeleistung und für weniger stark geschädigte Haare Produkte mit mäßiger oder geringer Pflegeleistung. Die Pflegeleistung kann durch bekannte Pflegestoffe wie quaternäre Stickstoffverbindungen (z.B. Hexadecyltrimethylammoniumchlorid), kationische Polymere (z.B. solche der INCI (International Nomenclature of Cosmetic Ingredients) Polyquaternium-10) oder Silikone (z.B. solche der INCI) erzeugt werden. Weiterhin sind auch insbesondere Dicarbonsäure (z.B. Bernsteinsäure) hierfür geeignet.

Die Ermittlung des Schädigungsgrads mit zugehöriger Produktempfehlung wird von der Datenverarbeitungseinrichtung 305 beispielsweise automatisch durchgeführt, z.B. durch eine Software, die auf der Datenverarbeitungseinrichtung, z.B. einem Smartphone, einem Tablets oder einem PC, installiert ist.

Die Datenverarbeitungseinrichtung 305 ermittelt das zu empfehlende Produkt beispielsweise basierend auf einer Datenbank, die für jeden Schädigungsgrad eine Produktempfehlung enthält. Ein Beispiel zeigt Tabelle 2.

**Tabelle 2**

| Schädigungsstufe | Produktempfehlung |
|---|---|
| Gering | Produkt 1 für geringe Haarpflege, enthaltend Hexadecyltrimethylammoniumchlorid (0,2%) |
| Mäßig | Produkt 2 für mäßige Haarpflege, enthaltend Hexadecyltrimethylammoniumchlorid (0,2%) + Polyquaternium-10 (0,5%) |
| Stark | Produkt 3 für starke Haarpflege, enthaltend Hexadecyltrimethylammoniumchlorid (0,2%) + Polyquaternium-10 (0,5%) + Dimethicon (1%) |
| Sehr stark | Produkt 4 für sehr starke Haarpflege, enthaltend Hexadecyltrimethylammoniumchlorid (0,2%) + Polyquaternium-10 (0,5%) + Dimethicon (1%) + Bernsteinsäure (1%) |

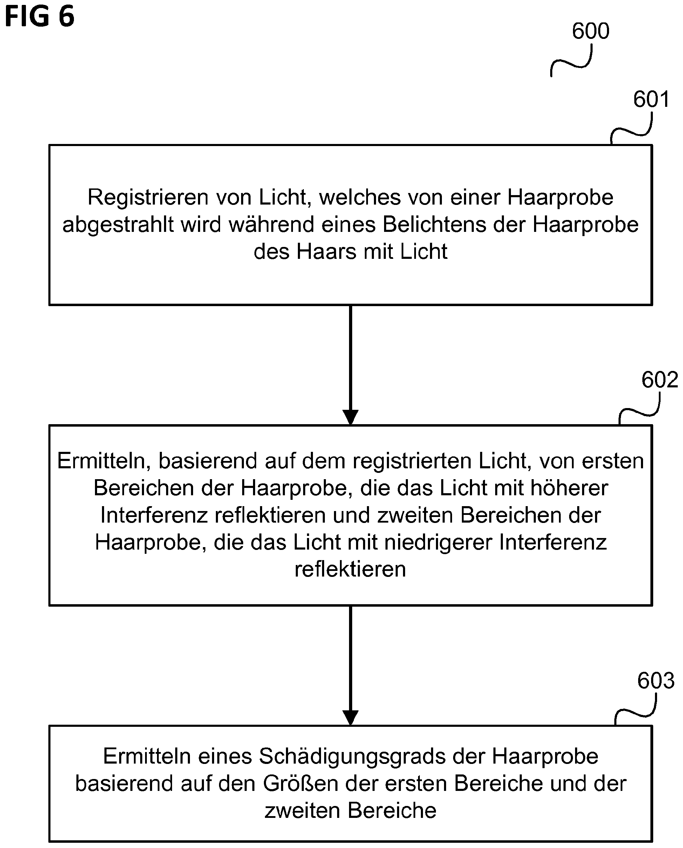

Zusammenfassend wird gemäß verschiedenen Ausführungsformen ein Verfahren zum Ermitteln eines Schädigungsgrads von Haar bereitgestellt, wie es in Figur 6 dargestellt ist.

Figur 6 zeigt ein Ablaufdiagramm 600.

In 601 wird während eines Belichtens einer Haarprobe des Haars mit Licht, Licht registriert, welches von der Haarprobe abgestrahlt wird.

In 602 werden basierend auf dem registrierten Licht erste Bereiche der Haarprobe, die das Licht mit höherer Interferenz reflektieren und zweite Bereiche der Haarprobe, die das Licht mit niedrigerer Interferenz reflektieren, ermittelt.

In 603 wird ein Schädigungsgrads der Haarprobe basierend auf den Größen der ersten Bereiche und der zweiten Bereiche (z.B. basierend auf der Gesamtgröße der ersten und/oder der Gesamtgröße der zweiten Bereiche, z.B. basierend auf dem Flächenanteil der Gesamtgröße der ersten Bereiche an der Gesamtfläche der Haarprobe oder basierend auf dem Verhältnis der Gesamtgröße der ersten Bereiche zur Gesamtgröße der zweiten Bereiche) ermittelt.

In anderen Worten wird gemäß verschiedenen Ausführungsformen ein Verfahren zur Ermittlung (und beispielsweise anschließende Verminderung) einer Haarschädigung bereitgestellt, die auf einer Analyse mit Hilfe von Interferenzmikroskopie aufgenommenen Mustern auf der Oberfläche ein oder mehrerer Haare basiert. Dabei wird die Stärke einer Schädigung eines Haars (oder mehrerer Haare) ermittelt, indem festgestellt wird, wie groß die Bereiche des Haares sind, in denen hohe Interferenz auftritt (beispielsweise im Verhältnis zu den Bereichen oder zum Gesamtbereich des beobachteten Haars). Eine hohe Interferenz kann beispielsweise dadurch auftreten, dass sich die Cuticula vom Kortex abhebt, was auf eine Schädigung des Haars hindeutet. Das Haar wird beispielsweise mit Weißlicht (z.B. Tageslicht) bestrahlt und das Auftreten von Interferenz in den verschiedenen Bereichen des Haars im reflektierten Licht untersucht.

Es sollte beachtet werden, dass auch das Ermitteln der Schädigung basierend auf dem Verhältnis der Größe der ersten Bereiche zur Gesamtfläche als auf der Größe der zweiten Bereiche basierend angesehen werden kann, da die Größe der ersten Bereiche desto geringer ist, je größer die zweiten Bereiche sind (die Summe der Größe der ersten Bereiche und der Größe der zweiten Bereiche ist beispielsweise die Gesamtfläche, gegebenenfalls zuzüglich von Bereichen, die weder den ersten Bereichen noch den zweiten Bereichen zugeordnet sind, beispielsweise Bereiche mittlerer Interferenz).

Das Verfahren gemäß Figur 6 kann mit einem tragbaren elektronischen Gerät wie beispielsweise einem Smartphone realisiert werden und schafft damit für einen Verbraucher eine einfache Möglichkeit zur Haarschädigungsbestimmung.

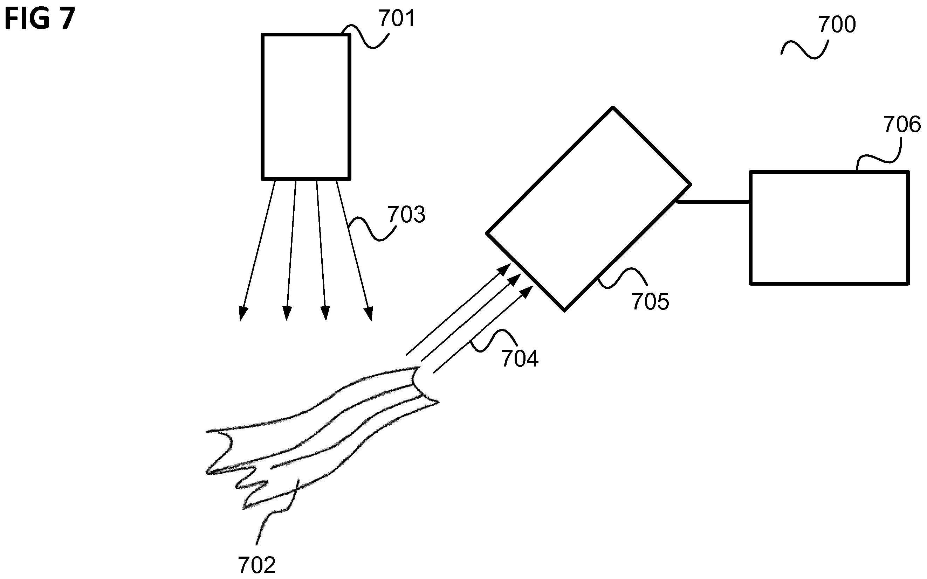

Das Verfahren gemäß Figur 6 wird beispielsweise durch eine Anordnung durchgeführt, wie sie in Figur 7 dargestellt ist.

Figur 7 zeigt eine Anordnung 700 zum Ermitteln eines Schädigungsgrads von Haar.

Die Anordnung 700 weist eine Lampe 701 auf, die eingerichtet ist, eine Haarprobe 702 des Haars mit Licht 703 zu belichten, und eine Lichtregistriervorrichtung 705, die eingerichtet ist, Licht 704, welches von der Haarprobe abgestrahlt wird, zu registrieren.

Die Anordnung weist ferner eine Datenverarbeitungseinrichtung 706 auf, die eingerichtet ist, basierend auf dem registrierten Licht erste Bereiche der Haarprobe, die das Licht mit höherer Interferenz reflektieren, und zweite Bereiche der Haarprobe, die das Licht mit niedrigerer Interferenz reflektieren, zu ermitteln und einen Schädigungsgrad der Haarprobe basierend auf der Größe der ersten Bereiche und der Größe der zweiten Bereiche zu ermitteln.

Es sollte beachtet werden, dass Ausführungsbeispiele, die im Zusammenhang mit dem Verfahren zum Ermitteln eines Schädigungsgrads von Haar analog für die Verfahren und Anordnung zum Ermitteln eines Schädigungsgrads von Haar gelten und umgekehrt.

Basierend auf dem ermittelten Schädigungsgrad können dem Verbraucher ferner (beispielsweise automatisch mittels seines Smartphones) eine objektiv ermittelte Empfehlung gegeben werden, welche Produkte für sein Haar gut geeignet sind, beispielsweise um der ermittelten Schädigung entgegenzuwirken.

Im Folgenden werden Ausführungsbeispiele angegeben:
Ausführungsbeispiel 1 ist ein Verfahren zum Ermitteln eines Schädigungsgrads von Haar, wie es in Figur 6 dargestellt ist.
Ausführungsbeispiel 2 ist ein Verfahren gemäß Ausführungsbeispiel 1, wobei das Registrieren des Lichts mittels eines Interferenzmikroskops erfolgt.
Ausführungsbeispiel 3 ist ein Verfahren gemäß Ausführungsbeispiel 1 oder 2, wobei das Registrieren des Lichts die Aufnahme eines Fotos der Haarprobe aufweist.
Ausführungsbeispiel 4 ist ein Verfahren gemäß einem der Ausführungsbeispiele 1 bis 3, wobei das Ermitteln der ersten Bereiche und das Ermitteln der zweiten Bereiche das Ermitteln von helleren Bereichen und dunkleren Bereichen des Fotos aufweist.
Ausführungsbeispiel 5 ist ein Verfahren gemäß einem der Ausführungsbeispiele 1 bis 4, wobei das Ermitteln der ersten Bereiche das Ermitteln von Bereichen des Fotos aufweist, deren Helligkeit größer als ein vorgegebener Schwellwert oder größer gleich einem vorgegebenen Schwellwert ist.
Ausführungsbeispiel 6 ist ein Verfahren gemäß Ausführungsbeispiel 5, wobei das Ermitteln der zweiten Bereiche das Ermitteln von Bereichen des Fotos aufweist, deren Helligkeit kleiner gleich dem vorgegebenen Schwellwert oder kleiner als der vorgegebene Schwellwert ist.
Ausführungsbeispiel 7 ist ein Verfahren gemäß einem der Ausführungsbeispiele 1 bis 6, aufweisend das Ermitteln des Schädigungsgrads für eine Vielzahl von Haarproben und Ermitteln, als den Schädigungsgrad des Haars, eines durchschnittlichen Schädigungsgrads der ermittelten Schädigungsgrade.
Ausführungsbeispiel 8 ist ein Verfahren gemäß einem der Ausführungsbeispiele 1 bis 7, wobei die Haarprobe ein oder mehrere Haare aufweist.
Ausführungsbeispiel 9 ist ein Verfahren gemäß einem der Ausführungsbeispiele 1 bis 8, wobei für das Ermitteln des Schädigungsgrads des Haars Ergebnisse von Vergleichsmessungen genutzt werden.
Ausführungsbeispiel 10 ist ein Verfahren gemäß einem der Ausführungsbeispiele 1 bis 9, wobei das Ermitteln des Schädigungsgrads basierend auf Kalibrierungsdaten erfolgt, die eine zuvor ermittelte Zuordnung von Anteilen von Bereichen höherer Interferenz von Haarproben zu Schädigungsgraden repräsentieren.
Ausführungsbeispiel 11 ist ein Verfahren gemäß Ausführungsbeispiel 10, aufweisend Ermitteln der Kalibrierungsdaten basierend auf Haarproben mit bekannter Schädigung.
Ausführungsbeispiel 12 ist ein Verfahren gemäß Ausführungsbeispiel 11, aufweisend Erzeugen der Haarproben bekannter Schädigung durch vorgegebenes Dehnen der Haare der Haarproben.
Ausführungsbeispiel 13 ist ein Verfahren gemäß einem der Ausführungsbeispiele 1 bis 12, aufweisend Belichten der Haarprobe mit Weißlicht.
Ausführungsbeispiel 14 ist ein Verfahren gemäß einem der Ausführungsbeispiele 1 bis 13, ferner aufweisend Auswählen eines nutzerspezifischen Mittels anhand des ermittelten Schädigungsgrades.
Ausführungsbeispiel 15 ist ein Verfahren gemäß Ausführungsbeispiel 14, ferner aufweisend Anzeigen des ausgewählten nutzerspezifischen Mittels.
Ausführungsbeispiel 16 ist eine Anordnung zum Ermitteln eines Schädigungsgrads von Haar, wie sie in Figur 7 dargestellt ist.

In einer weiteren Ausführungsform umfasst die Erfindung ein Verfahren zum Ermitteln einer individuellen Haarbehandlungsempfehlung gekennzeichnet durch die Schritte
a) Aufnahme von Interferenzmustern mehrerer Proben unterschiedlich geschädigter Haare;
b) Erstellen eines Kalibriermodells, das eine Korrelation zwischen Interferenzmustern und dem Schädigungsgrad herstellt;
c) Aufnahme von Interferenzmustern der Haare eines Individuums;
d) Bestimmung eines Schädigungsgrades der Haare dieses Individuums anhand des Kalibriermodells;
e) Ausgabe einer individuellen Behandlungsempfehlung zu den Haaren des Individuums in Abhängigkeit vom ermittelten Schädigungsgrad.

Es ist bevorzugt, dass die individuelle Behandlungsempfehlung die Empfehlung von Haarpflegeprodukten umfasst. Es kann bevorzugt sein, dass das Verfahren zum Ermitteln einer individuellen Haarbehandlungsempfehlung ferner einen Schritt zum Auslösen einer Bestellung eines empfohlenen, im Handel erhältlichen Haarpflegeprodukten umfasst. Es ist ferner bevorzugt, dass die individuelle Behandlungsempfehlung darin besteht, dem Individuum zum/vom Einsatz von Haarpflegeprodukten, die das Individuum anhand von QR-Codes, NFC-Chips, Barcodes oder RFID-Chips, identifiziert, zu- oder abzuraten.

Alternativ kann die individuelle Behandlungsempfehlung darin bestehen, dem Individuum zum Einsatz von Haarpflegeprodukten, die für das Individuum individuell hergestellt werden, zu zuraten und einen Bestellvorgang, vorzugsweise durch Aufrufen einer Internetseite eines Herstellers von individuellen Haarpflegeprodukten, einzuleiten.

Es ist ferner bevorzugt, das Kalibriermodell aus b) als gespeicherte Information auf einem lokalen Datenträger oder in einer Cloud vorliegt. Es ist auch bevorzugt, dass Schritt c) bei einem Friseur, an einem Verkaufspunkt ("point of sale", POS) von Haarbehandlungsmitteln oder im privaten Bereich durchgeführt wird.

Schritt c) wird vorzugsweise durch ein smartes Endgerät wie beispielsweise ein Smartphone, ein WLAN-fähiger Mediaplayer oder ein Tablet, vorzugsweise über eine vorab installierte App, gesteuert.

## Patentansprüche

1. Verfahren zum Ermitteln eines Schädigungsgrads von Haar (102), aufweisend:
während eines Belichtens einer Haarprobe (102P, 702) des Haars (102) mit Licht (703), Registrieren von Licht (704), welches von der Haarprobe (102P, 702) abgestrahlt wird;
wobei das verfahren dadurch charakterisiert ist, dass es ferner umfasst:
Ermitteln, basierend auf dem registrierten Licht (704), von ersten Bereichen der Haarprobe (102P, 702), die das Licht (704) mit höherer Interferenz reflektieren und zweiten Bereichen der Haarprobe (102P, 702), die das Licht (704) mit niedrigerer Interferenz reflektieren; und
Ermitteln eines Schädigungsgrads der Haarprobe (102P, 702) basierend auf den Größen der ersten Bereiche und der zweiten Bereiche, wobei der Schädigungsgrad basierend auf der Gesamtgröße der ersten und/oder der Gesamtgröße der zweiten Bereiche,
insbesondere basierend auf dem Flächenanteil der Gesamtgröße der ersten Bereiche an der Gesamtfläche der Haarprobe oder basierend auf dem Verhältnis der Gesamtgröße der ersten Bereiche zur Gesamtgröße der zweiten Bereiche erfolgt.

2. Verfahren gemäß Anspruch 1, wobei das Registrieren des Lichts (704) mittels eines Interferenzmikroskops (303) erfolgt.

3. Verfahren gemäß Anspruch 1 oder 2, wobei das Registrieren des Lichts (704) die Aufnahme eines Fotos der Haarprobe (102P, 702) aufweist.

4. Verfahren gemäß einem der Ansprüche 1 bis 3, wobei das Ermitteln der ersten Bereiche und das Ermitteln der zweiten Bereiche das Ermitteln von helleren Bereichen und dunkleren Bereichen des Fotos aufweist.

5. Verfahren gemäß einem der Ansprüche 1 bis 4, wobei das Ermitteln der ersten Bereiche das Ermitteln von Bereichen des Fotos aufweist, deren Helligkeit größer als ein vorgegebener Schwellwert oder größer gleich einem vorgegebenen Schwellwert ist.

6. Verfahren gemäß Anspruch 5, wobei das Ermitteln der zweiten Bereiche das Ermitteln von Bereichen des Fotos aufweist, deren Helligkeit kleiner gleich dem vorgegebenen Schwellwert oder kleiner als der vorgegebene Schwellwert ist.

7. Verfahren gemäß einem der Ansprüche 1 bis 6, aufweisend das Ermitteln des Schädigungsgrads für eine Vielzahl von Haarproben (102P, 702) und Ermitteln, als den Schädigungsgrad des Haars (102), eines durchschnittlichen Schädigungsgrads der ermittelten Schädigungsgrade.

8. Verfahren gemäß einem der Ansprüche 1 bis 7, wobei die Haarprobe (102P, 702) ein oder mehrere Haare (102) aufweist.

9. Verfahren gemäß einem der Ansprüche 1 bis 8, wobei für das Ermitteln des Schädigungsgrads des Haars (102) Ergebnisse von Vergleichsmessungen genutzt werden.

10. Verfahren gemäß einem der Ansprüche 1 bis 9, wobei das Ermitteln des Schädigungsgrads basierend auf Kalibrierungsdaten erfolgt, die eine zuvor ermittelte Zuordnung von Anteilen von Bereichen höherer Interferenz von Haarproben (102P, 702) zu Schädigungsgraden repräsentieren.

11. Verfahren gemäß Anspruch 10, aufweisend Ermitteln der Kalibrierungsdaten basierend auf Haarproben (102P, 702) mit bekannter Schädigung.

12. Verfahren gemäß Anspruch 11, aufweisend Erzeugen der Haarproben (102P, 702) bekannter Schädigung durch vorgegebenes Dehnen der Haare (102) der Haarproben (102P, 702).

13. Verfahren gemäß einem der Ansprüche 1 bis 12, aufweisend Belichten der Haarprobe (102P, 702) mit Weißlicht.

14. Verfahren zur individualisierten Haarbehandlung, **gekennzeichnet durch** die Schritte
a) Aufnahme von Interferenzmustern mehrerer Proben unterschiedlich geschädigter Haare (102);
b1) Erstellen eines Kalibriermodells, das eine Korrelation zwischen Interferenzmustern und dem Schädigungsgrad herstellt,
b2) Ermitteln eines Schädigungsgrades der mehreren Proben unterschiedlich geschädigter Haare (102) gemäß dem Verfahren gemäß nach Anspruch 1;
c) Aufnahme von Interferenzmustern der Haare (102) eines Individuums;
d) Bestimmung eines Schädigungsgrades der Haare (102) dieses Individuums anhand des Kalibriermodells;
e) Ausgabe einer individuellen Behandlungsanweisung zu den Haaren (102) des Individuums in Abhängigkeit vom ermittelten Schädigungsgrad.

15. Anordnung (300, 700) zum Ermitteln eines Schädigungsgrads von Haar (102), aufweisend:
Eine Lampe (701), die eingerichtet ist, eine Haarprobe (102P, 702) des Haars (102) mit Licht (703) zu belichten,
eine Lichtregistriervorrichtung (705, die eingerichtet ist, Licht (704), welches von der Haarprobe (102P, 702) abgestrahlt wird, zu registrieren; und wobei die Anordnung dadurch charakterisiert ist, dass die ferner
eine Datenverarbeitungseinrichtung (305, 706) umfasst, die eingerichtet ist,
basierend auf dem registrierten Licht (704) erste Bereichen der Haarprobe (102P, 702), die das Licht (704) mit höherer Interferenz reflektieren und zweite Bereiche der Haarprobe (102P, 702), die das Licht (704) mit niedrigerer Interferenz reflektieren, zu ermitteln und
einen Schädigungsgrad der Haarprobe (102P, 702) basierend auf der Größe der ersten Bereiche und der Größe der zweiten Bereiche zu ermitteln, wobei der Schädigungsgrad basierend auf der Gesamtgröße der ersten und/oder der Gesamtgröße der zweiten Bereiche, insbesondere basierend auf dem Flächenanteil der Gesamtgröße der ersten Bereiche an der Gesamtfläche der Haarprobe oder basierend auf dem Verhältnis der Gesamtgröße der ersten Bereiche zur Gesamtgröße der zweiten Bereiche erfolgt.

## Claims

1. A method for determining a degree of damage to hair (102), comprising:
during exposure of a hair sample (102P, 702) of the hair (102) to light (703), registering light (704) emitted by the hair sample (102P, 702); the method being **characterized in that** it further comprises:
determining, on the basis of the registered light (704), first regions of the hair sample (102P, 702) that reflect the light (704) with higher interference and second regions of the hair sample (102P, 702) that reflect the light (704) with lower interference; and determining a degree of damage to the hair sample (102P, 702) on the basis of the sizes of the first regions and the second regions, wherein the degree of damage is based on the total size of the first and/or the total size of the second regions, in particular based on the area proportion of the total size of the first regions to the total area of the hair sample or based on the ratio of the total size of the first regions to the total size of the second regions.

2. The method according to claim 1, wherein the light (704) is registered by means of an interference microscope (303).

3. The method according to claim 1 or 2, wherein registering the light (704) includes taking a photograph of the hair sample (102P, 702).

4. The method according to one of claims 1 to 3, wherein determining the first regions and determining the second regions includes determining lighter regions and darker regions of the photograph.

5. The method according to one of claims 1 to 4, wherein determining the first regions includes determining regions of the photograph of which the brightness is greater than a specified threshold value or greater than or equal to a specified threshold value.

6. The method according to claim 5, wherein determining the second regions includes determining regions of the photograph of which the brightness is less than or equal to the specified threshold value or less than the specified threshold value.

7. The method according to one of claims 1 to 6, comprising determining the degree of damage for a plurality of hair samples (102P, 702) and determining, as the degree of damage to the hair (102), an average degree of damage of the determined degrees of damage.

8. The method according to one of claims 1 to 7, wherein the hair sample (102P, 702) comprises one or more hairs (102).

9. The method according to one of claims 1 to 8, wherein results of comparative measurements are used to determine the degree of damage to the hair (102).

10. The method according to one of claims 1 to 9, wherein the degree of damage is determined on the basis of calibration data representing a previously determined allocation of proportions of regions of hair samples (102P, 702) with higher interference to degrees of damage.

11. The method according to claim 10, comprising determining the calibration data on the basis of hair samples (102P, 702) having known damage.

12. The method according to claim 11, comprising generating the hair samples (102P, 702) with known damage by specified stretching of the hair (102) of the hair samples (102P, 702).

13. The method according to one of claims 1 to 12, comprising exposing the hair sample (102P, 702) to white light.

14. A method for individualized hair treatment, **characterized by** the steps of
a) receiving interference patterns of multiple samples of hair (102) damaged to different degrees;
b1) creating a calibration model that establishes a correlation between interference patterns and the degree of damage,
b2) determining a degree of damage of the multiple samples of hair (102) damaged to different degrees according to the method according to according to claim 1;
c) receiving interference patterns of the hair (102) of an individual;
d) ascertaining a degree of damage to the hair (102) of this individual using the calibration model;
e) outputting individual treatment advice relating to the hair (102) of the individual according to the determined degree of damage.

15. An arrangement (300, 700) for determining a degree of damage to hair (102), comprising: a lamp (701) which is configured to expose a hair sample (102P, 702) of the hair (102) to light (703),
a light registering device (705) which is configured to register light (704) emitted by the hair sample (102P, 702); and the arrangement being **characterized in that** it further comprises
a data processing apparatus (305, 706) which is configured
to determine, on the basis of the registered light (704), first regions of the hair sample (102P, 702) that reflect the light (704) with higher interference and second regions of the hair sample (102P, 702) that reflect the light (704) with lower interference, and
to determine a degree of damage of the hair sample (102P, 702) on the basis of the size of the first regions and the size of the second regions, the degree of damage being based on the total size of the first and/or the total size of the second regions, in particular based on the area proportion of the total size of the first regions to the total area of the hair sample or based on the ratio of the total size of the first regions to the total size of the second regions.

## Revendications

1. Procédé pour la détermination d'un degré d'endommagement des cheveux (102), présentant : pendant l'exposition d'un échantillon de cheveux (102P, 702) des cheveux (102) à de la lumière (703), l'enregistrement de la lumière (704) émise par l'échantillon de cheveux (102P, 702) ; dans lequel le procédé est **caractérisé en ce qu'il** comprend en outre :
la détermination, sur la base de la lumière (704) enregistrée, de premières zones de l'échantillon de cheveux (102P, 702) qui réfléchissent la lumière (704) avec une interférence plus élevée et de secondes zones de l'échantillon de cheveux (102P, 702) qui réfléchissent la lumière (704) avec une interférence plus faible ; et la détermination d'un degré d'endommagement de l'échantillon de cheveux (102P, 702) sur la base des tailles des premières zones et des secondes zones, dans lequel le degré d'endommagement est effectué sur la base de la taille totale des premières zones et/ou sur la taille totale des secondes zones, en particulier sur la base de la proportion de surface de la taille totale des premières zones par rapport à la surface totale de l'échantillon de cheveux ou sur la base du rapport entre la taille totale des premières zones et la taille totale des secondes zones.

2. Procédé selon la revendication 1, dans lequel l'enregistrement de la lumière (704) est effectué au moyen d'un microscope interférentiel (303).

3. Procédé selon la revendication 1 ou 2, dans lequel l'enregistrement de la lumière (704) comprend la prise d'une photographie de l'échantillon de cheveux (102P, 702).

4. Procédé selon l'une des revendications 1 à 3, dans lequel la détermination des premières zones et la détermination des secondes zones présentent la détermination de zones plus claires et de zones plus sombres de la photographie.

5. Procédé selon l'une des revendications 1 à 4, dans lequel la détermination des premières zones présente la détermination de zones de la photographie dont la luminosité est supérieure à une valeur seuil prédéfinie ou supérieure ou égale à une valeur seuil prédéfinie.

6. Procédé selon la revendication 5, dans lequel la détermination des secondes zones présente la détermination de zones de la photographie dont la luminosité est inférieure ou égale à la valeur seuil prédéfinie ou inférieure à la valeur seuil prédéfinie.

7. Procédé selon l'une des revendications 1 à 6, présentant la détermination du degré d'endommagement pour une pluralité d'échantillons de cheveux (102P, 702) et la détermination, en tant que degré d'endommagement des cheveux (102), d'un degré d'endommagement moyen des degrés d'endommagement déterminés.

8. Procédé selon l'une des revendications 1 à 7, dans lequel l'échantillon de cheveux (102P, 702) présente un ou plusieurs cheveux (102).

9. Procédé selon l'une des revendications 1 à 8, dans lequel, pour la détermination du degré d'endommagement des cheveux (102), des résultats de mesures comparatives sont utilisés.

10. Procédé selon l'une des revendications 1 à 9, dans lequel la détermination du degré d'endommagement est effectuée sur la base de données d'étalonnage représentant une attribution, déterminée au préalable, de proportions de zones avec une interférence supérieure d'échantillons de cheveux (102P, 702) à des degrés d'endommagement.

11. Procédé selon la revendication 10, présentant la détermination des données d'étalonnage sur la base d'échantillons de cheveux (102P, 702) comportant un endommagement connu.

12. Procédé selon la revendication 11, présentant la production des échantillons de cheveux (102P, 702) comportant un endommagement connu par un étirement prédéfini des cheveux (102) des échantillons de cheveux (102P, 702).

13. Procédé selon l'une des revendications 1 à 12, présentant l'exposition de l'échantillon de cheveux (102P, 702) à une lumière blanche.

14. Procédé permettant le traitement capillaire individualisé, **caractérisé par** les étapes consistant à
a) enregistrer des motifs d'interférence de plusieurs échantillons de cheveux (102) endommagés différemment ;
b1) créer un modèle d'étalonnage établissant une corrélation entre les modèles d'interférence et le degré d'endommagement,
b2) déterminer un degré d'endommagement des échantillons de cheveux (102) endommagés différemment selon le procédé selon la revendication 1 ;
c) enregistrer des motifs d'interférence des cheveux (102) d'un individu ;
d) définir un degré d'endommagement des cheveux (102) dudit individu à l'aide du modèle d'étalonnage ;
e) délivrer en sortie une indication de traitement individuelle pour les cheveux (102) de l'individu en fonction du degré d'endommagement déterminé.

15. Agencement (300, 700) permettant de déterminer un degré d'endommagement des cheveux (102), présentant : une lampe (701) qui est configurée pour exposer un échantillon de cheveux (102P, 702) des cheveux (102) à de la lumière (703),
un dispositif d'enregistrement de lumière (705) qui est configuré pour enregistrer la lumière (704) émise par l'échantillon de cheveux (102P, 702) ; et dans lequel l'agencement est **caractérisé en ce qu'il** comprend en outre
un appareil de traitement de données (305, 706) qui est configuré pour
déterminer, sur la base de la lumière (704) enregistrée, des premières zones de l'échantillon de cheveux (102P, 702) qui réfléchissent la lumière (704) avec une interférence plus élevée et des secondes zones de l'échantillon de cheveux (102P, 702) qui réfléchissent la lumière (704) avec une interférence plus faible et
déterminer un degré d'endommagement de l'échantillon de cheveux (102P, 702) sur la base de la taille
des premières zones et de la taille des secondes zones, dans lequel le degré d'endommagement est effectué sur la base de la taille totale des premières zones et/ou de la taille totale des secondes zones, en particulier sur la base de la proportion de surface de la taille totale des premières zones par rapport à la surface totale de l'échantillon de cheveux ou sur la base du rapport entre la taille totale des premières zones et la taille totale des secondes zones.
